# EUROPEAN PATENT APPLICATION

(11) **EP 4 523 754 A2**
(43) Date of publication of application: **19.03.2025**
(21) Application number: 25153031.7
(22) Date of filing: 25.05.2020
(51) Int. Cl.: A61P 31/04

(54) **IMPROVED ADMINISTRATION OF GLYCYLCYCLINES**

(30) Priority: 24.05.2019 EP 19176477
(62) Divisional of application: 20729969.4
(71) Applicant: Stichting Radboud universitair medisch centrum, 6525 GA Nijmegen (NL)
(72) Inventor: VAN INGEN, Jakobus, 6531 SG NIJMEGEN (NL)
(74) Representative: Nederlandsch Octrooibureau

(57) **Abstract**

The present invention relates to a new administration regime for glycylcyclines. Inhalation of these antibiotics was found to potently treat infection by *Mycobacterium* microbes such as *Mycobacterium abscessus.*

## Description

### Field of the invention

The present invention relates to a new administration regime for glycylcyclines. Inhalation of these antibiotics was found to potently treat infection by *Mycobacterium* microbes such as *Mycobacterium abscessus.*

### Background art

Glycylcyclines, a relatively new group of antibacterial agents, have the central four-ring carbocyclic skeleton present in the tetracyclines that is necessary for antibacterial activity. In glycylcyclines, substitution of an N-alkyl-glycylamido group on the D ring at the 9th position facilitates the broader spectrum of activity (Sum et al., J. Med. Chem. 1994, 37, 184-188). This additionally creates the ability to overcome tetracycline resistance mechanisms (Pankey, J. Antimicrob. Chemother., 2005, 56, 470-480 doi:10.1093/jac/dki248), because glycylcyclines bind to the high-affinity tetracycline ribosomal binding site and evade Tet(M)- and Tet(O)-mediated ribosomal protection (Bergeron et al., Antimicrob. Agents Chemother., 1996, 40(9): 2226-2228). An example of a glycylcycline is tigecycline, which has a 9-*tert*-butyl-glycylamido side chain on the tetracycle, wherein the tetracycle in this case is minocycline. Other examples are eravacycline and DMG-DMDOT. Synthesis of glycylcyclines is known from EP0582790 and US5494903.

Glycylcyclines have a similar mechanism of antibiotic action as tetracyclines. They block protein synthesis, preventing bacterial reproduction. Both classes of antibiotics bind to the 30S ribosomal subunit to prevent the amino-acyl tRNA from binding to the A site of the ribosome. Glycylcyclines bind more effectively than tetracyclines. Glycylcyclines are active against other resistant pathogens including methicillin-resistant staphylococci, penicillin-resistant *Streptococcus pneumoniae,* and vancomycin-resistant enterococci. (Zhanel et al., Drugs (2004) 64: 63, doi.org/10.2165/00003495-200464010-00005)

Tigecycline is the only glycylcycline that is on the market for pharmaceutical use. It is only available as an injectable formulation, unlike tetracyclines, which can be available in oral dosage forms. Tigecycline has a significantly larger volume of distribution (>10 L/kg) than tetracyclines (range of 0.14to 1.6 L/kg). Protein binding is approximately 68%. Studies in rats using radiolabelled tigecycline demonstrated good penetration into tissues. Tigecycline has a half-life of 36 hours in humans, and not more than 15% of tigecycline is excreted unchanged in the urine. The pharmacokinetics of tigecycline are not markedly influenced by patient gender or age.

An important characteristic of tigecycline is that while it is safe, it leads to various side effects, particularly at doses required to achieve clinically relevant effects as known in the art. Common side effects of tigecycline include nausea and vomiting. In a study reported by Muralidharan (Antimicrob. Agents Chemother., 2005, 49 (1): 220-229. doi:10.1128/aac.49.1.220-229.2005) gastrointestinal adverse events were found to be dose limiting at 300 mg. In that study, the most common adverse events were nausea (33 of 68 tigecycline recipients [48.5%]) and vomiting (20 of 68 tigecycline recipients [29.4%]). CN103044281 discloses that improved purity of tigecycline can decrease the incidence of these side effects, but not eliminate it.

Related to drug resistance, one study evaluated dry powder inhalation formulation of 3-drug blends that sometimes comprised glycylcyclines. Different formulation blends based on colistin were developed and sometimes comprised tigecycline in a 3-drug combination further comprising either meropenem or rifampicin (Lee et al., J. Pharm. Sci., 2016, DOl: 10.1016/j.xphs.2016.02.007). The blends were designed to combine different mechanistic strategies for the constituting antibiotics. The blends were formulated for inhalation to avoid undesired precipitation of colistin in solutions. It was found to allow precise codelivery of the blend components. The blends were tested for in vitro activity at different particle sizes, and aerosolization of the developed dry powder was attempted in vitro. Antibiotic efficacy was tested against gram-negative bacteria using static in vitro time-kill assays applying a single concentration, and all tested colistin-comprising blends were found to be as bactericidally effective as colistin alone. WO2011073002 describes dry formulations for inhalation comprising particles of aminoglycoside antibiotics coated with magnesium stearate, for treating cystic fibrosis patients colonized with *Pseudomonas aeruginosa.*

Mycobacterial infections are known to be difficult to treat. The organisms are hardy because of their cell wall, which is neither truly Gram negative nor positive. Also, they are naturally resistant to a number of antibiotics that disrupt cell-wall biosynthesis, such as penicillin. Due to this particular cell wall, mycobacteria can survive long exposure to acids, alkalis, detergents, oxidative bursts, lysis by complement, and many antibiotics. Several mycobacteria are susceptible to the antibiotics clarithromycin and rifamycins such as amikacin, but antibiotic-resistant strains have emerged.

Of the mycobacteria, *Mycobacterium abscessus* is notoriously resistant to antibiotics. Ferro et al. (Antimicrob. Agents Chemother., 60:2895-2900. doi:10.1128/AAC.03112-15) describe a dose-response study in a hollow-fiber system model of pulmonary *M*. *abscessus* infection, using the injectable formulation. The optimal tigecycline clinical dose was identified as 200 mg/day. This dose exhibited a short-term inhibitory effect on *M*. *abscessus,* achieving 1-log kill. Ferro et al. also indicate that this dose will lead to adverse effects in virtually all patients, due to the associated plasma area under the concentration-time curve (AUC) of >6.87 mg, which is associated with nausea and vomiting. At higher doses a maximum microbial kill of 1.37 log₁₀CFU/mLwas identified.

In vivo treatment of *M*. *abscessus* is reported by Wallace et al. (J. Antimicrob. Chemother., 2014, doi:10.1093/jac/dku062), who collected data from patients treated with drug combinations including tigecycline. All patients received tigecycline at an initial intravenous dose of 100 mg, followed by 50 mg every 12 h administered over about 30-60 minutes. Additional standard concomitant medications including antibiotics were allowed. Tigecycline given for ≥1 month as part of a multidrug regimen resulted in improvement in about 60% of patients with *M*. *abscessus* and *M*. *chelonae* infections, including those with underlying cystic fibrosis, despite failure of prior antibiotic therapy. Efficacy outcome was assessed as improvement, failure, or indeterminate. Improvement was considered to have occurred when administration of tigecycline was associated with stabilization or improvement in signs and symptoms of the infection based on the information provided by the treating physicians. Adverse events were reported in about 90% of cases. Serious adverse events were reported in 29 out of 52 patients. The most common of these were exacerbation of the underlying condition, nausea, vomiting, sepsis/multiorgan failure, respiratory failure and pneumonia/respiratory infection. Fourteen of the 52 patients (27%) had to stop tigecycline therapy early (within the first month), because of adverse events.

In view of the ongoing arms race with drug resistant bacteria, there is an ongoing need for improved use of antibiotics. There is a need for improved administration regimes of glycylcyclines. Such improved regimes should preferably incur no or reduced adverse effects.

### Summary of the invention

It was surprisingly found that inhalation of glycylcycline led to unprecedented kill levels of gram positive bacteria, which are notoriously difficult to treat. In a first aspect, the invention provides a glycylcycline for use in the treatment of a subject infected with gram positive bacteria, wherein the glycylcycline is administered by inhalation. In preferred embodiments within this aspect the subject is infected with mycobacteria, preferably with nontuberculous mycobacteria. In more preferred embodiments the subject is infected with a mycobacterium of a clade selected from the group consisting of *M*. *abscessus, M*. *avium* complex, *M. tuberculosis* complex, *M. chelonae, M. fortuitum, M. mucogenicum, M. parafortuitum, M. simiae, M. kansasii,* and *M. peregrinum,* preferably of the clade *M*. *abscessus* or *M*. *avium* complex. In more preferred embodiments the subject is infected with a mycobacterium of the clade *M*. *abscessus,* or a subspecies thereof such as *M*. *abscessus* subspecies *abscessus, M*. *abscessus* subspecies *bolletii* or *M*. *abscessus* subspecies *massiliense.* Preferably the infection in the subject is a pulmonary infection. In preferred embodiments the subject is suffering from a disease selected from the group consisting of cystic fibrosis, non-cystic fibrosis bronchiectasis, pneumonia, chronic pulmonary obstructive disease, nontuberculous mycobacteria (NTM) pulmonary infection, and tuberculosis, preferably cystic fibrosis. In preferred embodiments the glycylcycline is tigecycline. Preferably, less than 400 mg/day is administered to a subject, more preferably less than 200 mg/day, even more preferably less than 100 mg/day. In preferred embodiments the treatment has at least 2 log kill efficacy. In preferred embodiments the glycylcycline is formulated as an inhalable formulation that further comprises a pharmaceutically acceptable excipient such as a stearate. In preferred embodiments the inhalable formulation comprises at least 80 wt.-% of glycylcycline. In preferred embodiments the inhalable formulation comprises glycylcycline microparticles having a volume diameter lower than 15 µm, preferably lower than 10 µm. In preferred embodiments, the glycylcycline is formulated for nebulisation or for dry powder inhalation, preferably for nebulisation. Preferably the administration of the glycylcycline is part of a multi-drug regimen for the treatment of the bacterial infection.

In another aspect of the invention is provided a method of treating infection with gram positive bacteria in a subject, the method comprising the step of administering glycylcycline to the subject via inhalation.

### Description of embodiments

In a first aspect the invention provides a glycylcycline for use in the treatment of a subject infected with gram positive bacteria, wherein the glycylcycline is administered by inhalation. Such a glycylcycline is referred to hereinafter as a glycylcycline for use according to the invention. In preferred embodiments the subject is a non-human subject. In other preferred embodiments the subject is a human subject. Preferably, the subject is a subject in need of treatment or at risk of infection, most preferably in need of treatment. Need of treatment can be need to cure an infection, but it can also be prophylactic treatment. In preferred embodiments, treatment is primary prophylactic treatment for the prevention of disease. The inventors found that the invention is also particularly suited for secondary prophylaxis, so in other preferred embodiments, treatment is secondary prophylactic treatment for the prevention of disease recurrence after earlier treatment. In preferred embodiments, a subject is a young subject, preferably a juvenile subject, more preferably a newborn subject. In other preferred embodiments, a subject is elderly. An elderly subject is preferably over 50 years of age, more preferably over 60, even more preferably over 65, more preferably still over 70, most preferably over 75.

### Gram positive bacteria

Gram positive bacteria are widely known in the art. Several genera are typically pathogenic in humans. Of these, *Streptococcus* and *Staphylococcus* are cocci (sphere-shaped). The remaining organisms are bacilli (rod-shaped) and can be subdivided based on their ability to form spores. The non-spore formers are *Corynebacterium* and *Listeria* (a coccobacillus), whereas *Bacillus* and *Clostridium* produce spores. The spore-forming bacteria can again be divided based on their respiration: *Bacillus* is a facultative anaerobe, while *Clostridium* is an obligate anaerobe.

In the context of this invention, gram positivity is intended to reflect the fact that gram staining leads to a stained bacterium. It is not intended to be limited to bacteria that have certain physical membrane characteristics (in general, the following characteristics are present in gram-positive bacteria: cytoplasmic lipid membrane; thick peptidoglycan layer; teichoic acids and lipoids are present, forming lipoteichoic acids, which serve as chelating agents, and also for certain types of adherence; peptidoglycan chains are cross-linked to form rigid cell walls by a bacterial enzyme DD-transpeptidase; a much smaller volume of periplasm than that in gram-negative bacteria; Slayer attached to the peptidoglycan layer; the presence of teichoic acids in the cell wall). Accordingly, the invention also encompasses glycyclcyclines for treatment of pseudo-gram-positive bacteria. For example, mycobacteria have a particular cell wall, which is neither truly Gram negative nor positive. Several species of mycobacteria stain gram positive, and are therefore encompassed by the invention. The staining behaviour of species is generally known (see for instance Pfyffer GE, Palicova F. Mycobacterium: General characteristics, laboratory detection, and staining procedures. In: Vesalovic et al (Eds). Manual of Clinical Microbiology, 10th Edition. ASM Press, 2011. pp. 472-502), and in any case can be ascertained by routine staining practice.

In preferred embodiments the invention provides the glycylcycline for use according to the invention, wherein the subject is infected with mycobacteria, preferably with nontuberculous mycobacteria. Mycobacteria form a genus of Actinobacteria, the Mycobacteriaceae. This genus includes pathogens known to cause serious diseases in mammals, including tuberculosis (*Mycobacterium tuberculosis*) and leprosy (*Mycobacterium leprae*) in humans. Mycobacteria are generally acid fast, and can be subdivided in *M. tuberculosis* complex (mycobacteria that can cause tuberculosis or leprosy) and in nontuberculous mycobacteria (the other mycobacteria, which can cause pulmonary disease resembling tuberculosis, lymphadenitis, skin disease, or disseminated disease). Preferably the mycobacteria are nontuberculous mycobacteria, as good results have been achieved in treating these gram positive bacteria. As is known in the art, not all mycobacteria stain positive under gram staining. Accordingly, preferred mycobacteria are gram positive mycobacteria.

In preferred embodiments is provided the glycylcycline for use according to the invention, wherein the subject is infected with a mycobacterium of a clade selected from the group consisting of *M*. *abscessus, M*. *avium* complex, *M. tuberculosis* complex, *M*. *chelonae, M. fortuitum, M. mucogenicum, M. parafortuitum, M. simiae, M. kansasii,* and *M. peregrinum,* preferably of the clade *M*. *abscessus* or *M*. *avium* complex. Mycobacteria of the *M*. *chelonae* clade are preferably selected from *M*. *chelonae, M. immunogenum,* and *M*. *stephanolepidis.* Mycobacteria of the *M*. *fortuitum* clade are preferably selected from *M. boenickei, M. brisbanense, M. cosmeticum, M. fortuitum, M. fortuitum* subsp. *acetamidolyticum, M. houstonense, M. mageritense, M. neworleansense, M. peregrinum, M. porcinum, M. senegalense,* and *M*. *septicum.* Mycobacteria of the *M*. *mucogenicum* clade are preferably selected from *M*. *aubagnese, M. mucogenicum,* and *M. phocaicum.* Mycobacteria of the *M. parafortuitum* clade are preferably selected from *M*. *austroafricanum, M. diernhoferi, M. frederiksbergense, M. hodleri, M. neoaurum,* and *M. parafortuitum.* Mycobacteria of the *M*. *simiae* clade are preferably selected from *M*. *florentinum, M. genavense, M. heidelbergense, M. interjectum, M. kubicae, M. lentiflavum, M. montefiorense, M. palustre, M. parascrofulaceum, M. simiae,* and *M*. *triplex.* Mycobacteria of the *M*. *kansasii* clade are preferably selected from *M*. *gastri* and *M*. *kansasii.* Mycobacteria of the *M*. *avium* complex are preferably selected from *M*. *avium, M. avium paratuberculosis* (which has been implicated in Crohn's disease in humans and is the causative agent of Johne's disease in cattle and sheep), *M*. *avium silvaticum, M. avium "hominissuis", M. avium avium, M. colombiense, M. indicus pranii, M. chimaera, M. vulneris, M. arosiense, M. bouchedurhonense, M. timonense, M. marseillense, M. yongonense, M. paraintracellulare* and *M. intracellulare.* A preferred gram positive mycobacterium is *M. peregrinum.* Mycobacteria of the *M*. *abscessus* clade are preferably selected from *M*. *abscessus* subspecies *abscessus, M*. *abscessus* subspecies *bolletii* or *M*. *abscessus* subspecies *massiliense.*

In more preferred embodiments is provided the glycylcycline for use according to the invention, wherein the subject is infected with a mycobacterium of the clade *M*. *abscessus,* or a subspecies thereof such as *M*. *abscessus* subspecies *abscessus, M*. *abscessus* subspecies *bolletii* or *M*. *abscessus* subspecies *massiliense.*

### Treatment

A glycylcycline for use according to the invention is for use in the treatment of a subject infected with gram positive bacteria, wherein the glycylcycline is administered by inhalation. In the context of this invention, treatment of a bacterial infection may refer to preventing, ameliorating, curing, and/or delaying an infection with gram positive bacteria. Prevention of an infection may mean that an infection is cured before symptoms of the infection have manifested. Preferably, successful treatment may mean that:
- The severity of at least one symptom of the infection has been reduced, and/or
- A symptom has not manifested while this manifestation could have been expected, and/or
- At least a parameter associated with the infection has been improved.

Preferred symptoms are selected from the group consisting of epithelial destruction, redness, edema, haemorrhage, exudate, cough, sputum production, fatigue, weight loss, malaise, night sweats, fever, chest pain, decreased exercise tolerance, dyspnoea, and dizziness, more preferably selected from cough, sputum production, fatigue, weight loss, malaise, night sweats, and fever.

A parameter may be a symptom as described above, or it may preferably be selected from the group consisting of prolongation of patient survival, improvement of the quality of life, observed pain relief, amelioration of a comorbid condition, decrease in the number of live mycobacteria in respiratory samples measured by quantitative cultures, decrease in the number of live mycobacteria in respiratory samples measured by semi-quantitative cultures, decrease in the grade of sputum smear positivity by direct microscopy and auramine or Ziehl-Neelsen staining, no live mycobacteria detectable by culture in respiratory samples, improvement of lesions seen on computed tomography scans of the lungs, increase in forced expiratory volume in 1 second (FEV1) in pulmonary function testing, increased exercise tolerance, increased walking distance in the 6-minute walk test, and improvement in quality of life reported in St Georges Respiratory Questionnaire or optionally other quality of life measurement. More preferably a symptom is selected from the group consisting of prolongation of patient survival, decrease in the number of live mycobacteria in respiratory samples measured by quantitative cultures, decrease in the grade of sputum smear positivity by direct microscopy and auramine or Ziehl-Neelsen staining, no live mycobacteria detectable by culture in respiratory samples, improvement of lesions seen on computed tomography scans of the lungs, increase in forced expiratory volume in 1 second (FEV1) in pulmonary function testing, increased walking distance in the 6-minute walk test, and improvement in quality of life reported in St Georges Respiratory Questionnaire.

In the context of the invention, treating, preventing, curing and/or delaying an infection is preferably assessed or detected after at least one day, two days, three days, four days, one week, two weeks, three weeks, four weeks, one month, two months, three months, four months, five months, six months or more in a treated subject. Treating, preventing, curing and/or delaying an infection is preferably identified in a subject as:
- a prolongation of patient survival of at least one month, several months or more (compared to those not treated or treated with a control or compared with the subject at the onset of the treatment) and/or
- improvement of the quality of life and observed symptom relief such as pain relief.

In the context of the invention, a patient may survive and/or may be considered as being disease free. Alternatively, the infection may have been stopped or delayed. In the context of the invention, an improvement of quality of life and observed pain relief may mean that a patient may need less pain relief drugs than at the onset of the treatment. Alternatively or in combination with the consumption of less pain relief drugs, a patient may be less short of breath than at the onset of the treatment. "Less" in this context may mean 5% less, 10% less, 20% less, 30% less, 40% less, 50% less, 60% less, 70% less, 80% less, 90% less. A patient may no longer need any pain relief drug or respiratory assistance. This improvement of quality of life and observed pain relief may be seen, detected or assessed after at least one week, two weeks, three weeks, four weeks, one month, two months, three months, four months, five months, six months or more of treatment in a patient and compared to the quality of life and observed pain relief at the onset of the treatment of said patient.

Preferably the infection is an opportunistic infection. In preferred embodiments is provided the glycylcycline for use according to the invention, wherein the subject is infected with a pulmonary infection. Pulmonary infection can be any infection of the respiratory tract, including infections that contribute to tissue damage. This pulmonary infection is preferably by the gram positive bacteria described earlier herein. Preferred pulmonary infections are upper respiratory tract infection such as nasal cavity infection, frontal sinus infection, maxillary sinus infection, pharynx infection, or larynx infection; and lower respiratory tract infection, such as tracheal infection, bronchial infection, endobronchial infection, bronchiectasis infection, bronchiolar infection, alveolar infection, lung parenchymal infection, and lung infection. Most preferred infections are bronchial infection, endobronchial infection, bronchiectasis infection, bronchiolar infection, alveolar infection, lung parenchymal infection, and lung infection

As a result of treatment, a subject may be cured of all pulmonary infection. In this case it can be said that the lungs of the subject have been sterilised. Preferably at least part of the infecting gram positive bacteria are killed, more preferably all infecting gram positive bacteria are killed.

The reduction of micro-organisms is conventionally classified using a logarithmic scale. A single log reduction is a 90% reduction of organisms, and is often referred to as a 1 log kill. A two log reduction is a 99% reduction of organisms, referred to as a 2 log kill, followed by a three log reduction (99.9%, referred to as 3 log kill), etc. Preferably the treatment has at least 1 log kill efficacy. In preferred embodiments is provided the glycylcycline for use according to the invention, wherein the treatment has at least 2 log kill efficacy. More preferably, the treatment has 3 log kill efficacy. Even more preferably, the treatment has 3.5 log kill efficacy, more preferably still 4 log kill efficacy, even more preferably still 4.5 log kill efficacy, most preferably 5 log kill efficacy. Log kill efficacy can be determined using techniques known in the art, for example by bacteria count or CFU determination, preferably as described in the examples. Efficacy is preferably assessed after 1 week of treatment, more preferably after 2, 3, 4, 5, 6, 7, 8, 9, 10, 11, or 12 weeks of treatment, even more preferably after 4 weeks of treatment. Such treatment preferably involves administration by inhalation 4, 3, 2, or 1 times per day, or 7, 6, 5, 4, 3, 2, or 1 times per week. Preferably, efficacy is assessed by culture such as sputum culture after at least four weeks and preferably after at least eight weeks of treatment wherein treatment consisted of daily administration of at least 1.25 mg glycylcycline for use according to the invention, more preferably as exemplified in Example 1. Sputum culture as described herein is preferably sputum culture using Middlebrook 7H9 liquid medium, Middlebrook 7H10 solid medium, Middlebrook 7H11 solid medium, Ogawa solid medium, Coletsos solid medium, Stonebrink solid medium, or Lowenstein-Jensen solid medium, optionally with other terms and definitions preferably as described in Example 1.

In preferred embodiments, efficacy is classified using smear microscopy with subsequent grading (1+, 2+, 3+, 4+). Herein, sputum samples are analysed by direct microscopy with Ziehl-Neelsen staining or auramine staining to visualize the bacteria. The density of mycobacteria is assessed by the number of mycobacteria seen per visual field and graded based thereupon, applying the World Health Organisation-approved grading system (WHO reference number: WHO/HTM/TB/2015.11 ).

In preferred embodiments, efficacy is classified using semi-quantitative culture, preferably sputum culture, measuring time-to-positivity. Herein, the time it takes from the moment of incubation of the liquid culture to the moment the liquid culture presents enough growth to activate its sensor and be reported by the automated liquid culture monitoring device, is monitored. This time-to-positivity is a marker for the number of viable bacteria present in the clinical sample. Successful treatment preferably reduces the amount of live bacteria.

In preferred embodiments, the glycylcycline according to the invention does not cause adverse effects.

Treatment using glycylcycline for use according to the invention preferably comprises administration 6 times per day, more preferably 5, 4, 3, 2, or 1 time per day, even more preferably 1 time per day. In further preferred embodiments, administration is less than one time per day such as 6, 5, 4, 3, 2, or 1 times per week.

Treatment using glycylcycline for use according to the invention preferably comprises administration of at least 0.5 mg of glycylcycline, more preferably at least 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 21, 22, 23 ,24, 25, 30, 35, 40, 45, 50, 60, 70, 80, 90, 100, 110, 120, 130, 140, 150, 160, 170, 180, 190, or 200 mg per administration, even more preferably at least 10 mg, more preferably still at least 50 mg, even more preferably 60 mg, more preferably 70 mg, more preferably still 80 mg, even more preferably at least 100 mg, more preferably still at least 125 mg, most preferably at least 150 mg per administration. In other preferred embodiments treatment using glycylcycline for use according to the invention preferably comprises administration of at least 300 mg of glycylcycline, more preferably at least 400, even more preferably at least 500 mg, most preferably at least 600 mg. Treatment using glycylcycline for use according to the invention preferably comprises administration of at most 1000 mg of glycylcycline, more preferably at most 900, 800, 700, 600, 500, 400, 350, 300, 250, 240, 230, 220, 210, 200, 190, 180, 170, 160, 150, 140, 130, 120, 110, or 100 mg per administration, even more preferably at most 600 mg, more preferably still at most 500 mg, even more preferably still at most 400 mg, even more preferably 200 mg, more preferably still at most 150 mg, even more preferably at most 140 mg, more preferably 130 mg, more preferably still 120 mg, more preferably still 110 mg, even more preferably at most 100 mg, more preferably 90 mg, more preferably still at most 80 mg, most preferably at most 75 mg per administration. In preferred embodiments is provided the glycylcycline for use according to the invention, wherein less than 400 mg/day is administered to a subject, preferably less than 200 mg/day, more preferably less than 100 mg/day. Mention of a dose per administration is preferably the dose that is administered in a single inhalation or in a single consecutive series of inhalation, preferably in a single inhalation. In other preferred embodiments a dose is the total cumulative dose that is administered over one day.

In preferred embodiments is provided the glycylcycline for use according to the invention, wherein the subject is suffering from a disease selected from the group consisting of cystic fibrosis, non-cystic fibrosis bronchiectasis, pneumonia (such as preferably hospital-acquired pneumonia or ventilator-acquired pneumonia), chronic pulmonary obstructive disease, nontuberculous mycobacteria (NTM) pulmonary infection, and tuberculosis, preferably cystic fibrosis. Cystic fibrosis, also known as CF, mucovoidosis or mucoviscidosis, is a fatal genetic disorder. Although technically it is considered a rare disease, cystic fibrosis is ranked as one of the most widespread life-shortening genetic diseases and it affects more than 60,000 people worldwide. It is associated with impairment in the transport of chloride ions across the epithelial membranes of exocrine glands, which causes a decreased water content of their secretions. Morphological changes of dilation and hypertrophy of the bronchial glands are followed by mucous plugging. Said viscid mucus in the airways allows bacterial colonization, with consequent infection of the respiratory tract, contributing to ongoing tissue damage. *Haemophilus influenza* and *Staphylococcus aureus* are the first pathogens that colonize the airway in childhood. As the lung disease progresses, colonization by the pathogens such as *Pseudomonas aeruginosa* will follow. After a period of intermittent colonization with *Pseudomonas aeruginosa,* the colonization becomes chronic in most CF patients, and virtually impossible to be eradicated. Nontuberculous mycobacteria are increasingly recognized as causative agents of such opportunistic infections in humans. Pulmonary infections are most frequent. *Mycobacterium avium* complex and *Mycobacterium abscessus* cause 70% and 20% of these pulmonary infections, depending on geographical location. *Mycobacterium abscessus* specifically infects patients with Cystic Fibrosis. Infections by *Mycobacterium abscessus* are the most feared mycobacterial infections in humans, because they are virtually untreatable with current antibiotics. In preferred embodiments the glycylcycline for use according to the invention is for treating infection with *Mycobacterium abscessus* in a subject suffering from cystic fibrosis. In preferred embodiments the glycylcycline for use according to the invention is for treating infection in a subject suffering from hospital-acquired pneumonia or ventilator-acquired pneumonia.

In preferred embodiments the glycylcycline for use according to the invention is for preventing infection by gram positive bacteria, preferably by mycobacteria. Such prevention is preferably in subjects suffering from cystic fibrosis, or in subjects who have recently been cured from an infection, preferably from a gram positive bacterial infection such as a pulmonary gram positive bacterial infection, more preferably from a mycobacterial pulmonary infection.

Treatment as described herein is advantageously combined with further treatment, for example in multi-drug regimens. Such multi-drug regimens comprise simultaneous or sequential administration of at least two different drugs. This helps prevent the rise of drug resistance in bacteria (Lee et al., J. Pharm. Sci., 2016, DOl: 10.1016/j.xphs.2016.02.007). In preferred embodiments is provided the glycylcycline for use according to the invention, wherein administration of the glycylcycline is part of a multi-drug regimen for the treatment of the bacterial infection. Preferred multi-drug regimens involve further use of colistin, meropenem, aminoglycosides such as tobramycin, or rifampicin, preferably of at least two of colistin, meropenem, aminoglycosides, or rifampicin. Other preferred multi-drug regimens involve further use of imipenem, cefoxitin, aminoglycosides such as amikacin, macrolides such as azithromycin and clarithromycin or clofazimine, preferably of at least two of imipenem, cefoxitin, aminoglycosides, or macrolides.

### Method of treatment

In another aspect, the invention provides a method of treating, preventing, or delaying infection with gram positive bacteria in a subject in need thereof, the method comprising the step of administering glycylcycline to the subject via inhalation. In preferred embodiments, an effective amount is administered, which is an amount that provides a beneficial effect. More features and definitions have been provided elsewhere herein.

Treatment as described herein can be understood as the manufacture of a medicament for treatment of the indicated condition.

### Formulation

The glycylcycline for use according to the invention is administered by inhalation, and is therefore preferably formulated for inhalation. Administration of particles by inhalation to the respiratory system can be by means such as known in the art. For example, particles or agglomerates can be delivered from an inhalation device. In a preferred embodiment, particles are administered via a dry powder inhaler (DPI). Metered-dose-inhalers (MDI), aerosol spray inhalers, nebulizers, or instillation techniques also can be employed. Preferably, delivery is to the bronchioli and alveoli region of the pulmonary system, the central airways, or the upper airways, most preferably to bronchiole, lung tissue and alveolar macrophages.

Various suitable devices and methods of inhalation which can be used to administer particles to a patient's respiratory tract are known in the art. For example, suitable inhalers are described in US4995385, US4069819, and US5997848. Other examples include, but are not limited to, the Spinhaler(R) (Fisons, Loughborough, U.K.), Rotahaler(R) (Glaxo-Wellcome, Research Triangle Technology Park, North Carolina), FlowCaps(R) (Hovione, Loures, Portugal), Inhalator.RTM. (Boehringer-Ingelheim, Germany), the ;Aerolizer(R) (Novartis, Switzerland), the diskhaler (Glaxo-Wellcome, RTP, NC) and others, known to those skilled in the art, such as aerosol spray inhalers.

In preferred embodiments the formulations are administered as a dry powder via a dry powder inhaler. In one embodiment, the dry powder inhaler is a simple, breath actuated device. An example of a suitable inhaler which can be employed is described in US6766799. A receptacle is used to enclose or store particles and/or respirable pharmaceutical compositions comprising the particles for subsequent administration. The receptacle is filled with the particles using methods as known in the art. For example, vacuum filling or tamping technologies may be used. Generally, filling the receptacle with the particles can be carried out by methods known in the art. In one embodiment, the particles that are enclosed or stored in a receptacle have a mass of at least about 5 milligrams up to about 100 milligrams, in another embodiment, the mass of the particles stored or enclosed in the receptacle comprises a mass of bioactive agent from at least about 1.5 mg to at least about 20 milligrams. In one embodiment, the volume of the inhaler receptacle is at least about 0.37 cm³ to 0.95 cm³. Alternatively, the receptacles can be capsules or blisters. Suitable capsules can be obtained, for example, from Shionogi (Rockville, Md.). ;Blisters can be obtained, for example, from Hueck Foils, (Wall, N.J.). Other receptacles and other volumes thereof suitable for use in the instant invention are also known to those skilled in the art. ;Preferably, particles administered to the respiratory tract travel through the upper airways (oropharynx and larynx), the lower airways which include the trachea followed by bifurcations into the bronchi and bronchioli and through the terminal bronchioli which in turn divide into respiratory bronchioli leading then to the ultimate respiratory zone, the alveoli or the deep lung. In a preferred embodiment, most of the mass of particles deposits in the deep lung. In another embodiment, delivery is primarily to the central airways. Delivery to the upper airways can also be obtained. Aerosol dosage, formulations and delivery systems also may be selected for a particular therapeutic application, as described, for example, in Gonda, I. "Aerosols for delivery of therapeutic and diagnostic agents to the respiratory tract," in Critical Reviews in Therapeutic Drug Carrier Systems, 6: 273-313, 1990; and in Moren, "Aerosol dosage forms and formulations," in: Aerosols in Medicine. Principles, Diagnosis and Therapy, Moren et al., Eds, Elsevier, Amsterdam, 1985.

The glycylcycline for use according to the invention is preferably comprised in a composition. In preferred embodiments is provided the glycylcycline for use according to the invention, wherein the glycylcycline is formulated as an inhalable formulation that further comprises a pharmaceutically acceptable excipient such as a lubricant, preferably a stearate. A preferred stearate is a stearate salt of a bivalent pharmaceutically acceptable metal ion such as magnesium. Formulation of medicaments, ways of administration, and the use of pharmaceutically acceptable excipients are known and customary in the art and for instance described in Remington; The Science and Practice of Pharmacy, 21st Edition 2005, University of Sciences in Philadelphia.

In preferred embodiments is provided the glycylcycline for use according to the invention, wherein the inhalable formulation comprises at least 1 wt.-%, preferably at least 10 wt.-%, more preferably at least 80 wt.-% of glycylcycline. More preferably, at least 90, 95, 96, 97, 98, or 99 wt.-% is comprised.

In preferred embodiments is provided the glycylcycline for use according to the invention, wherein the formulation further comprises additional pharmaceutically active agents, preferably further antibiotics, more preferably at least one agent selected from colistin, meropenem, aminoglycosides such as tobramycin, or rifampicin, preferably of at least two of colistin, meropenem, aminoglycosides, or rifampicin.

In preferred embodiments is provided the glycylcycline for use according to the invention, wherein the glycylcycline is formulated for nebulisation or for dry powder inhalation, preferably for nebulisation.

In preferred embodiments, the glycylcycline is formulated as a dry powder formulation for pulmonary administration, such as granulated drug powder, liposomal formulation, or mechano-fused microparticles. Preferred forms comprise mechano-fused microparticles consisting of particles of glycylcycline in an amount comprised between 98 and 99.9% w/w and a lubricant such as magnesium stearate in an amount comprised between 0.1 and 2% w/w, said lubricant (preferably magnesium stearate) forming a coating of the whole surface of the drug particles for at least 50%, wherein at least 90% of said microparticles have a volume diameter lower than about 10 micron, said microparticles being obtainable by a mechano-fusion process. WO2011073002 describes suitable methods for preparing coated particles by such a process. The term "coat" preferably means that a lubricant such as magnesium stearate forms a film around the active particles. The coating is only partial when the amount of lubricant is not sufficient for forming a film around the whole surface of all the particles of the active ingredient.

The resulting smoothed and lubricated surfaces appear to reduce inter-particular forces (intermolecular surface forces and frictional forces) within the powder, thus giving rise to better dispersion performance during aerosolization. Mechano-fused microparticles may be crystalline or amorphous. The percentage of amorphicity, expressed as weight percent with respect to the total weight of the microparticles, may greatly vary and it may be equal to or higher than 50%, preferably of at least 70%, even more preferably of at least 90%. Said percentage of amorphicity may be determined using X-ray powder diffraction or other known techniques known to the skilled person such as differential scanning calorimelry (DSC) or microcalorimetry.

In preferred embodiments, the glycylcycline is in a liposomal formulation. The liposomal formulation preferably is formed into respirable aggregates for administration by inhalation. Any liposomal formulation technique known in the art may be used. Liposomal formulations which may be utilized may include a glycylcycline prepared as a first phase, optionally in combination with a solubilizer, while a second phase is prepared containing at least one phospholipid. The two phases may be combined, thereby forming liposomes comprising the glycylcycline. As noted above, these liposomes may then be formed into respirable aggregates and administered by inhalation.

In embodiments, a composition of the present disclosure may include at least one respirable aggregate including a liposome comprising a glycylcycline; and, at least one phospholipid such as lecithin, lysolecithin, phosphatidylcholine, phosphatidylethanolamine, phosphatidylinositol, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG- phosphatidylethanolamine, PVP-phosphatidylethanolamine, and combinations thereof, wherein the respirable aggregate has a mass median aerodynamic diameter of from about 1 µm to about 5 µm.

Methods which may be utilized for forming such respirable liposomal aggregates include, but are not limited to, controlled precipitation, evaporative precipitation into aqueous solution, spray freezing into liquid, ultra-rapid freezing, high internal phase emulsion processes, combinations thereof, and the like. In embodiments, a method may include preparing a first phase including a glycylcycline, optionally in combination with a solubilizer; preparing a second phase including at least one phospholipid; contacting the first phase with the second phase to form liposomes comprising the glycylcycline; recovering the liposomes; and forming the liposomes into respirable aggregates having a mass median aerodynamic diameter of from about 1 µm to about 5 µm. This is a suitable size for drops that are delivered by a nebulizer or pressurized metered dose inhaler, or dry powder for a dry powder inhaler, such drops or powders including the aggregates and particles. Methods and techniques for preparing dried liposomal formulations are known in the art, for example as disclosed in EP2227085.

Inhalable formulations can be formulated for nebulisation, preferably for administration using a nebulizer. A nebulizer is well known in the art, and is a drug delivery device used to administer medication in the form of a mist inhaled into the lungs. Nebulizers are commonly used for the treatment of cystic fibrosis. The lung deposition characteristics and efficacy of a generated aerosol depends largely on the particle or droplet size. Generally, the smaller the particle the greater its chance of peripheral penetration and retention. However, for very fine particles below 0.5 µm in diameter there is a chance of avoiding deposition altogether and being exhaled. In general, particles of more than 10 µm in diameter are most likely to deposit in the mouth and throat, for those of 5-10 µm diameter a transition from mouth to airway deposition occurs, and particles smaller than 5 µm in diameter deposit more frequently in the lower airways and are highly appropriate for pharmaceutical aerosols. Suitable nebulisers and excipients for formulations for nebulisation according to this invention are disclosed in US2017296562.

In general terms, the particle size of particles is quantified by measuring a characteristic equivalent sphere diameter, known as volume diameter, by laser diffraction. The particle size can also be quantified by measuring the mass diameter by means of suitable instruments well known to the skilled person. The volume diameter (VD) is related to the mass diameter (MD) by the density of the particles (assuming a size independent density for the particles). The term "mechano-fused" refers to microparticles constituted of two different materials wherein a first material is mechanically fused onto a second by a dry process. Particle size is preferably expressed in terms of volume diameter and the particle size distribution is expressed in terms of: i) the volume median diameter (VMD) which corresponds to the diameter of 50 percent by weight or volume respectively, of the particles, and ii) the volume diameter (VD) in micron of 10% and 90% of the particles, respectively.

Particles can be "agglomerated", which is used herein with two different meanings. The term "agglomerated microparticles" refers to particles which consist of more than one microparticles, those microparticles being adhered to each other. For example, an agglomerated microparticle of of 1.5 micron may consist of a large number of microparticles each having a lower diameter, adhered together. On the contrary, the term "loose-agglomerated microparticles" refers to particles in form of soft agglomerates which could easily break-up to give rise to the single microparticles.

Formulations, particularly dry powder formulations, preferably have suitable flow properties. The term "suitable flow properties" refers to a formulation that is easy handled during the manufacturing process and is able of ensuring an accurate and reproducible delivering of the therapeutically effective dose. The expression "accurate therapeutically active dose of the active ingredient" refers to a formulation wherein, upon actuation, the mean emitted dose is equal to or higher than 60% of the nominal dose, preferably higher than 65%, even more preferably higher than 70%.

Formulations are preferably chemically stable and/or physically stable. The expression "chemically stable" refers to a formulation that, upon storage, meets the requirements of the EMEA Guideline CPMP/QWP/122/02 referring to "Stability Testing of Existing Active Substances and Related Finished Products". The expression "physically stable" refers to a formulation that does not change its physical state in the device before use and upon storage.

The expression 'respirable fraction' refers to an index of the percentage of active particles which would reach the deep lungs in a patient. The respirable fraction, also termed fine particle fraction (FPF), is evaluated using a suitable in vitro apparatus, typically the Multistage Cascade Impactor or Multi Stage Liquid Impinger (MLSI) according to procedures reported in common Pharmacopoeias. However other apparatus such as Twin Stage Apparatus may be advantageously used.

When formulations are used in treatment, they are preferably used in a therapeutically effective amount. The term "therapeutically effective amount" preferably means the amount of the glycylcycline, that, when delivered to the lungs via a formulation such as a dry powder formulation as described herein, provides the desired biological effect.

In preferred embodiments is provided the glycylcycline for use according to the invention, wherein the inhalable formulation comprises glycylcycline microparticles having a volume diameter lower than 15 µm, preferably lower than 10 µm. The particle size of the microparticles of the invention is preferably lower than 15 micron. Advantageously, at least 90% of the particles have a volume diameter lower than about 10 micron. More advantageously no more than 10% of the microparticles have a volume diameter lower than 0.1 micron. Preferably no more than 50% of particles have a volume diameter lower than 0.6, and preferably it is comprised between 0.7 and 2.0 micron, more preferably between 0.8 and 1.5 micron. Particle size can be measured by laser diffraction according to known methods. Microparticles and mechano-fused microparticles of the invention may be present in the form of unagglomerated, e.g., individual, or stable agglomerated microparticles, those stable agglomerated microparticles having a particle size comprised in one of the ranges described above. The microparticles of the invention exhibit a very low amount of residual water, e.g. preferably lower than 5.0% w/w, more preferably comprised between 4.8% and 4.5% w/w, as determined according to known methods such as the Karl-Fisher method.

The invention also provides a capsule for use with a dry powder inhaler filled with the glycylcycline for use according to the invention, or with a formulation as described herein. Dry powder inhalers can be divided into two basic types:
i) single dose inhalers, for the administration of single subdivided doses of the active compound; each single dose is usually filled in a capsule;
ii) multidose dry powder inhalers, pre-loaded with quantities of active principles sufficient for longer treatment cycles.

### Glycylcyclines

Glycylcyclines are a class of antibacterial agents that is known in the art. They feature a carbocyclic skeleton that is also present in the tetracyclines that is necessary for antibacterial activity. In glycylcyclines, substitution of an N-alkyl-glycylamido group on the D ring at the 9th position facilitates the broader spectrum of activity (Sum et al., J. Med. Chem. 1994, 37, 184-188).

In preferred embodiments the glycylcycline is of general formula 1: 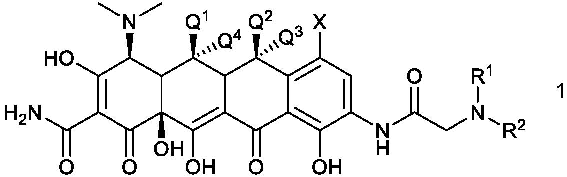 wherein
Q¹, Q², Q³, and Q⁴ are each independently chosen from -H, -CH₃, and -OH;
R¹ and R² are each independently chosen from H or C₁₋₆ hydrocarbon, or R¹ and R² together with the N to which they are attached form a 3- to 7-membered cyclic structure;
X is selected from -H, -halogen, or -N(R¹¹)(R²²); and
R¹¹ and R²² are each independently chosen from H or C₁₋₆ hydrocarbon, or R¹¹ and R²² together with the N to which they are attached form a 3- to 7-membered cyclic structure. More preferably, the glycylcycline is of general formula 1a: 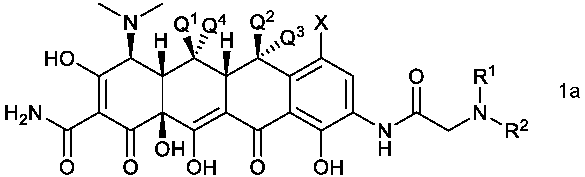
Q¹, Q², Q³, and Q⁴ are each independently chosen from -H, -CH₃, and -OH; in preferred embodiments, Q³ and Q⁴ are -H; in more preferred embodiments, Q¹ is -OH or -H, Q² is -CH₃ or-H, Q³ is -H or -OH, and Q⁴ is -H; in even more preferred embodiments, each of Q¹, Q², Q³, and Q⁴ are -H, or Q¹ is -OH, Q² is -CH₃, Q³ is -H, and Q⁴ is -H, or Q¹ is -H, Q² is -CH₃, Q³ is -OH, and Q⁴ is -H; when any of Q¹, Q², Q³, or Q⁴ is not -H, X is preferably H; in highly preferred embodiments, each of Q¹, Q², Q³, and Q⁴ are -H; such a glycylcycline is of general formula 2: 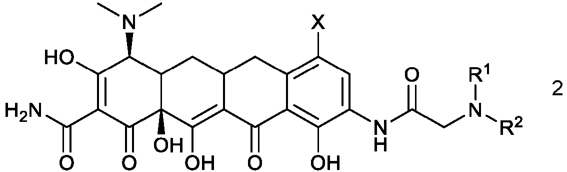 wherein
   R¹ and R² are each independently chosen from H or C₁₋₆ hydrocarbon, or R¹ and R² together with the N to which they are attached form a 3- to 7-membered cyclic structure;
   X is selected from -H, -halogen, or -N(R¹¹)(R²²); and
   R¹¹ and R²² are each independently chosen from H or C₁₋₆ hydrocarbon, or R¹¹ and R²² together with the N to which they are attached form a 3- to 7-membered cyclic structure. It is highly preferable that for glycylcyclines of general formula 2 the stereochemistry is as in general formula 2a: 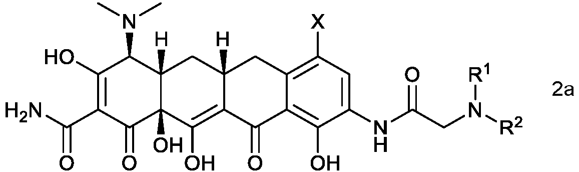
   R¹ and R² are each independently chosen from H or C₁₋₆ hydrocarbon, or R¹ and R² together with the N to which they are attached form a 3- to 7-membered cyclic structure. C₁₋₆ hydrocarbon can be unsaturated, and is preferably selected from C₁₋₆ alkyl, C₂₋₆ alkenyl, and C₂₋₆ alkynyl. C₁₋₆ alkyl is an even more preferred C₁₋₆ hydrocarbon and can be methyl, ethyl, *n*-propyl, isopropyl, cyclopropyl, *n*-butyl, *sec*-butyl, *tert*-butyl, isobutyl, cyclobutyl, *n*-pentyl, *tert*-pentyl, neopentyl, pentan-2-yl, pentan-3-yl, sec-isopentyl, 2-methylbutyl, and 3-methylbutyl, cyclopentyl, *n*-hexyl, and cyclohexyl. In preferred embodiments the C₁₋₆ hydrocarbon is a C₁₋₄ alkyl. R¹ and R² can form a cyclic structure with the N to which they are connected. In preferred embodiments this cyclic structure is 4, 5, or 6-membered, most preferably it is 5-membered, forming a pyrrolyl moiety. In preferred embodiments, R¹ and R² are both methyl, or R¹ and R² together form a 3- to 7-membered cyclic structure that is preferably pyrrolyl, or R¹ is H and R² is C₄ alkyl that is preferably *tert*-butyl. Most preferably R¹ is H and R² is *tert*-butyl.
   X is selected from -H, -halogen, or -N(R¹¹)(R²²); when any of Q¹, Q², Q³, or Q⁴ is not -H, X is preferably H; when X is -halogen, it is preferably -F; when each of Q¹, Q², Q³, or Q⁴ is-H, X is preferably halogen or -N(R¹¹)(R²²), more preferably -F or-Cl or -N(R¹¹)(R²²), even more preferably -F or -N(R¹¹)(R²²), most preferably -N(R¹¹)(R²²). X is preferably halogen or -N(R¹¹)(R²²), more preferably -F or -Cl or -N(R¹¹)(R²²), even more preferably -F or -N(R¹¹)(R²²), most preferably-N(R¹¹)(R²²).
   R¹¹ and R²² are each independently chosen from H or C₁₋₆ hydrocarbon, or R¹¹ and R²² together with the N to which they are attached form a 3- to 7-membered cyclic structure. C₁₋₆ hydrocarbon can be unsaturated, and is preferably selected from C₁₋₆ alkyl, C₂₋₆ alkenyl, and C₂₋₆ alkynyl. C₁₋₆ alkyl is an even more preferred C₁₋₆ hydrocarbon and can be methyl, ethyl, *n*-propyl, isopropyl, cyclopropyl, *n*-butyl, *sec*-butyl, *tert*-butyl, isobutyl, cyclobutyl, *n*-pentyl, *tert*-pentyl, neopentyl, pentan-2-yl, pentan-3-yl, sec-isopentyl, 2-methylbutyl, and 3-methylbutyl, cyclopentyl, *n-*hexyl, and cyclohexyl. In preferred embodiments the C₁₋₆ hydrocarbon is a C₁₋₄ alkyl. R¹¹ and R²² can form a cyclic structure with the N to which they are connected. In preferred embodiments this cyclic structure is 4, 5, or 6-membered, most preferably it is 5-membered, forming a pyrrolyl moiety. In preferred embodiments, R¹¹ and R²² are both methyl, or R¹¹ and R²² together form a 3- to 7-membered cyclic structure that is preferably pyrrolyl, or R¹¹ is H and R²² is C₄ alkyl that is preferably tert-butyl. Most preferably, R¹¹ and R²² are both methyl.

In preferred embodiments, halogen is selected from F, Cl, or Br, more preferably from F or Cl. Most preferably, halogen is F.

In preferred embodiments, the glycylcycline is selected from the group consisting of tigecycline, eravacycline, DMG-DMDOT, DMG-MINO, and DMG-DOXY, more preferably selected from the group consisting of tigecycline, eravacycline, DMG-DMDOT, and DMG-MINO. Most preferably the glycylcycline is tigecycline. Accordingly, in preferred embodiments is provided the glycylcycline for use according to the invention, wherein the glycylcycline is tigecycline. These preferred glycylcyclines are shown below with their structures above their names.

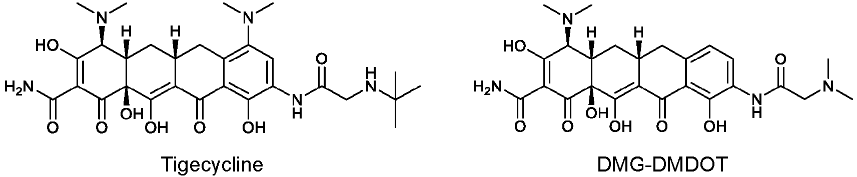

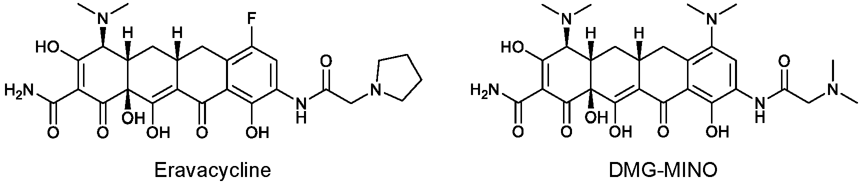

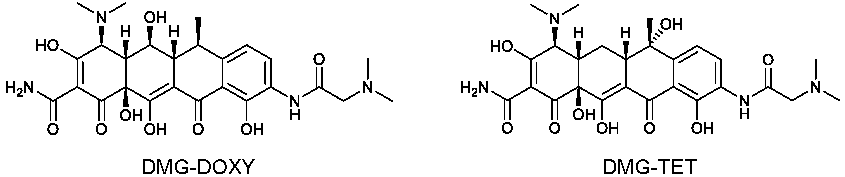

Synthesis of glycylcyclines is known from EP0582790, US5494903, and Sum et al., J. Med. Chem. 1994, 37, 184-188, amongst various other sources.

In preferred embodiments an aminomethylcycline is used instead of a glycylcycline. Aminomethylcyclines are known in the art, and preferred aminomethylcyclines are the compounds of which the molecular structures are shown in tables 2 and 3 of Honeyman et al., Antimicrob. Agents Chemother., 2015 (59):11. 7044-7053 (DOI:10.1128/AAC.01536-15 ). All features and definitions that do not define the molecular structure of a glycylcycline also apply to aminomethylcyclines. Aminomethylcyclines share a close structural relationship with glycylcyclines. The mechanism of action of aminomethylcyclines is similar to that of other tetracyclines and glycylcyclines, namely inhibition of bacterial protein synthesis. They share activity against bacterial strains expressing the two main forms of tetracycline resistance (efflux and ribosomal protection, see Draper et al., Antimicrob. Agents Chemother. 58 (3): 1279-1283. doi:10.1128/AAC.01066-13). Activity of aminomethylcyclines against mycobacteria is known (Shoen et al., 2019, Antimicrob. Agents Chemother., DOI: 10.1128/AAC.02522-18 ; Kaushik et al., 2019, Antimicrob. Agents Chemother., DOI: 10.1128/AAC.00470-19 which also demonstrates Eravacycline activity). More preferred aminomethylcyclines are compounds 4, 6, 26, 8, 27, 28, 29, 9, 30, 31, 23, 32, 33, 7, 34, 35, 36, 10, 37, 38, 11, 39, 40, 41, and 42 in table 3 of Honeyman et al., cited above. A most preferred aminomethylcycline is omadacycline (CAS: 389139-89-3 , compound 27 of Honeyman et al.)

### General definitions

Each embodiment as identified herein may be combined together unless otherwise indicated. All patent and literature references cited in the present specification are hereby incorporated by reference in their entirety.

When a structural formula or chemical name is understood by the skilled person to have chiral centers, yet no chirality is indicated, for each chiral center individual reference is made to all three of either the racemic mixture (having any enantiomeric excess), the pure R enantiomer, and the pure S enantiomer.

In the context of this invention, a decrease or increase of a parameter to be assessed means a change of at least 5% of the value corresponding to that parameter. More preferably, a decrease or increase of the value means a change of at least 10%, even more preferably at least 20%, at least 30%, at least 40%, at least 50%, at least 70%, at least 90%, or 100%. In this latter case, it can be the case that there is no longer a detectable value associated with the parameter.

The use of a compound or composition as a medicament as described in this document can also be interpreted as the use of said compound or composition in the manufacture of a medicament. Similarly, whenever a compound or composition is used for as a medicament, it can also be used for the manufacture of a medicament, or in a method.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value (of 10) more or less 1% of the value.

In the context of this invention, a cell or a sample can be a cell or a sample from a sample obtained from a subject. Such an obtained sample can be a sample that has been previously obtained from a subject. Such a sample can be obtained from a human subject. Such a sample can be obtained from a non-human subject.

### Particular embodiments

1. A glycylcycline for use in the treatment of a subject infected with gram positive bacteria, wherein the glycylcycline is administered by inhalation.
2. The glycylcycline for use according to embodiment 1, wherein the subject is infected with mycobacteria, preferably with nontuberculous mycobacteria.
3. The glycylcycline for use according to embodiment 1 or 2, wherein the subject is infected with a mycobacterium of a clade selected from the group consisting of *M*. *abscessus, M*. *avium* complex, *M. tuberculosis* complex, *M*. *chelonae, M. fortuitum, M. mucogenicum, M. parafortuitum, M. simiae, M. kansasii,* and *M. peregrinum,* preferably of the clade *M*. *abscessus* or *M*. *avium* complex.
4. The glycylcycline for use according to any one of embodiments 1-3, wherein the subject is infected with a mycobacterium of the clade *M*. *abscessus,* or a subspecies thereof such as *M*. *abscessus* subspecies *abscessus, M*. *abscessus* subspecies *bolletii* or *M*. *abscessus* subspecies *massiliense.*
5. The glycylcycline for use according to any one of embodiments 1-4, wherein the subject is infected with a pulmonary infection.
6. The glycylcycline for use according to any one of embodiments 1-5, wherein the subject is suffering from a disease selected from the group consisting of cystic fibrosis, non-cystic fibrosis bronchiectasis, pneumonia, chronic pulmonary obstructive disease, nontuberculous mycobacteria (NTM) pulmonary infection, and tuberculosis, preferably cystic fibrosis.
7. The glycylcycline for use according to any one of embodiments 1-6, wherein the glycylcycline is tigecycline.
8. The glycylcycline for use according to any one of embodiments 1-7, wherein less than 400 mg/day is administered to a subject, preferably less than 200 mg/day, more preferably less than 100 mg/day.
9. The glycylcycline for use according to any one of embodiments 1-8, wherein the treatment has at least 2 log kill efficacy.
10. The glycylcycline for use according to any one of embodiments 1-9, wherein the glycylcycline is formulated as an inhalable formulation that further comprises a pharmaceutically acceptable excipient such as a stearate.
11. The glycylcycline for use according to embodiment 10, wherein the inhalable formulation comprises at least 80 wt.-% of glycylcycline.
12. The glycylcycline for use according to embodiments 10 or 11, wherein the inhalable formulation comprises glycylcycline microparticles having a volume diameter lower than 15 µm, preferably lower than 10 µm.
13. The glycylcycline for use according to any one of embodiments 1-12, wherein the glycylcycline is formulated for nebulisation or for dry powder inhalation, preferably for nebulisation.
14. The glycylcycline for use according to any one of embodiments 1-13, wherein administration of the glycylcycline is part of a multi-drug regimen for the treatment of the bacterial infection.
15. Method of treating infection with gram positive bacteria in a subject, the method comprising the step of administering glycylcycline to the subject via inhalation.

### Examples

### Example 1

GM-CSF KO (granulocyte-macrophage colony-stimulating factor knockout) mice were infected through the intrapulmonary aerosol route using 1×10⁶ cfu/50uL of *M*. *abscessus.* Bacteria were delivered in a saline solution as 50µL doses via an FMJ-250 high-pressure syringe device (PennCentury) with an attached MicroSprayer (MicroSprayer, model IA-C; PennCentury, Philadelphia, PA, USA). To confirm bacterial uptake, 3 mice were sacrificed within the first 24 hours of bacteria exposure and the lungs were removed for colony forming units (CFU) determination on Middlebrook 7H11 media supplemented OADC (oleic albumin dextrose catalase) with carbenicillin and cycloheximide.

Tigecycline was reconstituted in 300µL of 0.9% endotoxin-free saline solution (TekNova) immediately before administration as to maximize bactericidal effects, and the mice received the dose variant TGC via intrapulmonary aerosol delivery using the same MicroSprayer device and methodology described above. All of the treatment groups (5 mice per group) received one 50µL dose 5 days a week for 4 consecutive weeks. Tigecycline was administered in three different doses, amounting to 2.5 mg/dose (2 groups, i.e. 10 mice in total), 1.25 mg/dose and 0.25 mg/dose.

After 4 weeks of treatment, mice were sacrificed and the lungs were removed for CFU determination on Middlebrook 7H11 media supplemented with OADC, carbenicillin, and cycloheximide. The result of CFU counts are presented in Table 1.

**Table 1: Mycobacterium abscessus CFU counts after 4 weeks of inhaled tigecycline treatment**

| | **Log10 CFU count** | |
|---|---|---|
| | ***At start*** | ***After 4 weeks*** |
| **Untreated** | 4.80 | 3.93 |
| **Inhaled tigecycline 0.25 mg** | 4.80 | 3.28 |
| **Inhaled tigecycline 1.25 mg** | 4.80 | 0.695 |
| **Inhaled tigecycline 2.5 mg** | 4.80 | 0.50 |

For the 2.5 mg cohorts, no bacterial colonies were visible after culturing lung tissue from 9 out of 10 mice treated with tigecycline for 4 weeks; one mouse suffered from abscess formation leading to incomplete response, with 0.5 Iog10 CFU count results.

## Claims

1. A glycylcycline for use in the treatment of a subject infected with gram positive bacteria, wherein the glycylcycline is administered by inhalation,
wherein the glycylcycline is tigecycline,
wherein the subject is infected with a pulmonary infection.

2. The glycylcycline for use according to claim 1, wherein the subject is infected with mycobacteria.

3. The glycylcycline for use according to claim 1, wherein the subject is infected with nontuberculous mycobacteria.

4. The glycylcycline for use according to any one of claims 1-3, wherein the subject is infected with a mycobacterium of a clade selected from the group consisting of *M*. *abscessus, M*. *avium* complex, *M*. *tuberculosis* complex, *M*. *chelonae, M. fortuitum, M. mucogenicum, M. parafortuitum, M. simiae, M. kansasii,* and *M. peregrinum,* preferably of the clade *M*. *abscessus* or *M*. *avium* complex.

5. The glycylcycline for use according to any one of claims 1-4, wherein the subject is infected with a mycobacterium of the clade *M*. *abscessus,* or a subspecies thereof such as *M*. *abscessus* subspecies *abscessus, M*. *abscessus* subspecies *bolletii* or *M*. *abscessus* subspecies *massiliense.*

6. The glycylcycline for use according to any one of claims 1-5, wherein the subject is suffering from a disease selected from the group consisting of cystic fibrosis, non-cystic fibrosis bronchiectasis, pneumonia, chronic pulmonary obstructive disease, nontuberculous mycobacteria (NTM) pulmonary infection, and tuberculosis, preferably cystic fibrosis.

7. The glycylcycline for use according to any one of claims 1-5, wherein the subject is suffering from cystic fibrosis.

8. The glycylcycline for use according to any one of claims 1-7, wherein less than 400 mg/day is administered to a subject, preferably less than 200 mg/day, more preferably less than 100 mg/day.

9. The glycylcycline for use according to any one of claims 1-8, wherein the treatment has at least 2 log kill efficacy.

10. The glycylcycline for use according to any one of claims 1-9, wherein the glycylcycline is formulated as an inhalable formulation that further comprises a pharmaceutically acceptable excipient such as a stearate.

11. The glycylcycline for use according to claim 10, wherein the inhalable formulation comprises at least 80 wt.-% of glycylcycline.

12. The glycylcycline for use according to claims 10 or 11, wherein the inhalable formulation comprises glycylcycline microparticles having a volume diameter lower than 15 µm, preferably lower than 10 µm.

13. The glycylcycline for use according to any one of claims 1-12, wherein the glycylcycline is formulated for nebulisation or for dry powder inhalation, preferably for nebulisation.

14. The glycylcycline for use according to any one of claims 1-13, wherein administration of the glycylcycline is part of a multi-drug regimen for the treatment of the bacterial infection.

15. Method of treating infection with gram positive bacteria in a subject, the method comprising the step of administering glycylcycline to the subject via inhalation.
